# EUROPEAN PATENT APPLICATION

(11) **EP 2 273 285 A1**
(43) Date of publication of application: **12.01.2011**
(21) Application number: 08752337.9
(22) Date of filing: 02.05.2008
(51) Int. Cl.: G01T 1/161

(54) **OPEN TYPE PET EQUIPMENT**

(71) Applicant: National Institute of Radiological Sciences, Chiba-shi Chiba 263-8555 (JP)
(72) Inventor: YAMAYA, Taiga, Chiba-shi Chiba 263-8555 (JP); MURAYAMA, Hideo, Chiba-shi Chiba 263-8555 (JP); MORI, Shinichirou, Chiba-shi Chiba 263-8555 (JP)
(74) Representative: Twelmeier Mommer & Partner
(86) International application number: PCT/JP2008/058432
(87) International publication number: WO 2009/133628

(57) **Abstract**

In an open-type PET scanner including a plurality of detector rings having multiple rings arrayed in the body axis direction, radiation measurement is performed while at least one detector ring is relatively moved with respect to a subject in the body axis direction, thereby dispersing simultaneous radiation in an open region to suppress a local reduction in sensitivity. The detector rings are optimized in constitution, moving direction and/or moving speed, thus making it possible to reduce the variation of distribution of sensitivity and expand a clearance in the open region and a field-of-view in the body axis direction.

## Description

### Technical Field

The present invention relates to an open-type PET scanner which includes a plurality of detector rings having multiple rings. The present invention relates in particular to an open-type PET scanner capable of suppressing a local reduction in sensitivity as well as expanding an open-region clearance between the detector rings and a field-of-view in the body axis direction, without increasing the number of detectors.

### Background Art

Positron emission tomography (PET) has gained attention as being effective in making an early diagnosis of cancers, cerebrovascular disorders, dementia and others. PET is a method for injecting a compound labeled with a trace amount of a positron emission nuclide to detect annihilation radiation emitted from the body, thereby imaging of metabolic functions such as sugar metabolism and examining the presence or absence of a disease and the seriousness of a disease. For the implementation thereof, PET scanners have been put into practical use.

The principle of PET is as follows. Positrons emitted from a positron emission nuclide by the positron decay undergo pair annihilation with electrons in the vicinity, and the thus generated a pair annihilation radiation at 511 keV is determined by a pair of radiation detectors according to the principle of coincidence. Thereby, the position at which the nuclide is present can be localized on one line segment (coincidence line) connecting between the pair of detectors. When an axis from the head of a patient to the feet is defined as a body axis, a distribution of the nuclide on a planar surface intersecting perpendicular with the body axis is obtained by image reconstruction in two-dimensional mode from data of the coincidence line determined on the planar surface from several directions.

Therefore, earlier PET scanners were constituted with single ring-type detectors in which detectors were densely arranged on a planar surface which was given as a field-of-view in such a manner so as to surround a field-of-view in a ring shape. Thereafter, with the advent of a multiple ring-type detector in which many single ring-type detectors were densely arranged in the body axis direction, a field-of-view in two-dimensional mode was changed to that in three-dimensional mode. Then, in the 1990s, 3-D mode PET scanners were developed one after another in which the coincidence was also determined between detector rings to increase the sensitivity greatly. This trend is found even now.

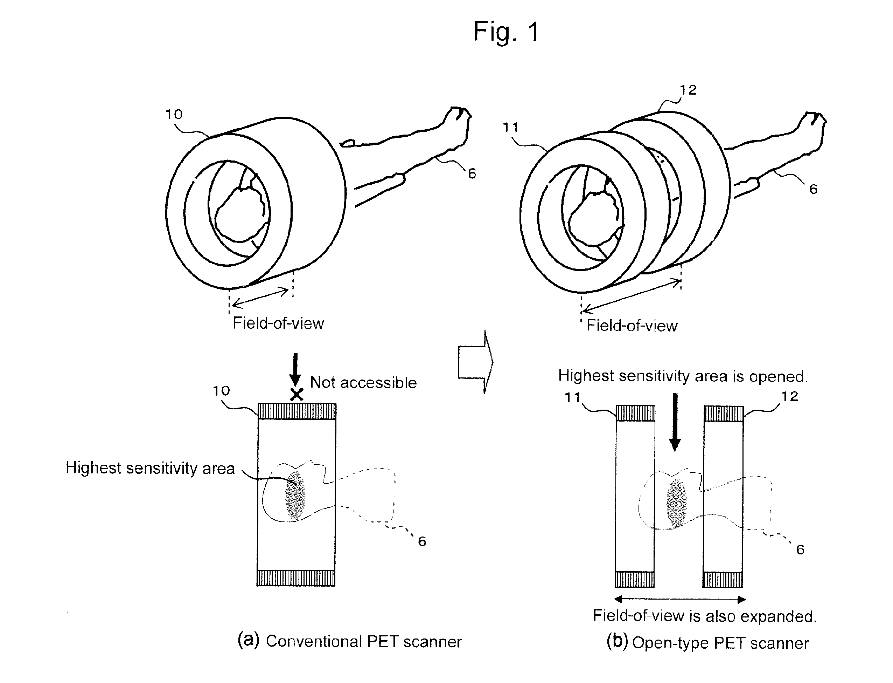

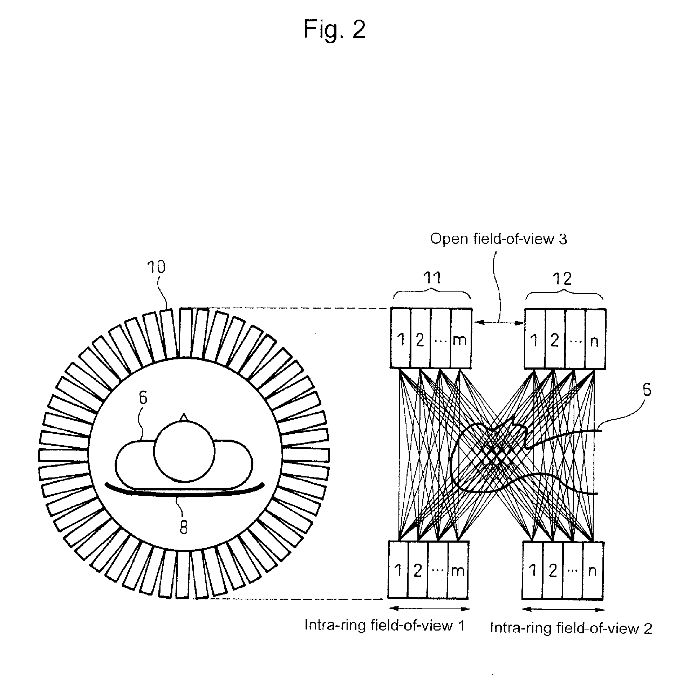

In order to increase the sensitivity of a PET scanner, as illustrated in Fig. 1(a), it is necessary to increase a solid angle by arranging densely detectors in a tunnel shape to constitute a multiple ring-type detector 10. However, a long tunnel-shaped patient port not only causes increased psychological stress to a patient 6 under examination but also affects medical care of the patient. In order to cope with this problem, as illustrated in Fig. 1(b), the applicant has proposed an open-type PET scanner in which multiple ring-type detectors 11, 12 which have been divided into plural regions in the body axis direction of the patient 6 are arranged apart to have a field-of-view region which is physically opened (also referred to as an open visual region). In an open region, as shown in Fig. 2, an image is reconstructed from remaining coincidence lines between the multiple ring-type detectors 11, 12. In this drawing, the reference numeral 8 depicts a bed.

As shown in Fig. 1(b) and Fig. 2, there has been so far designed an open-type PET scanner in which a detector is divided into two parts equal in width (refer to Taiga Yamaya, Taku Inaniwa, Shinichi Minohara, Eiji Yoshida, Naoko Inadama, Fumihiko Nishikido, Kengo Shibuya, Chih Fung Lam and Hideo Murayama, "A proposal of an open PET geometry," Phy. Med. Biol., 53, pp. 757-773, 2008).

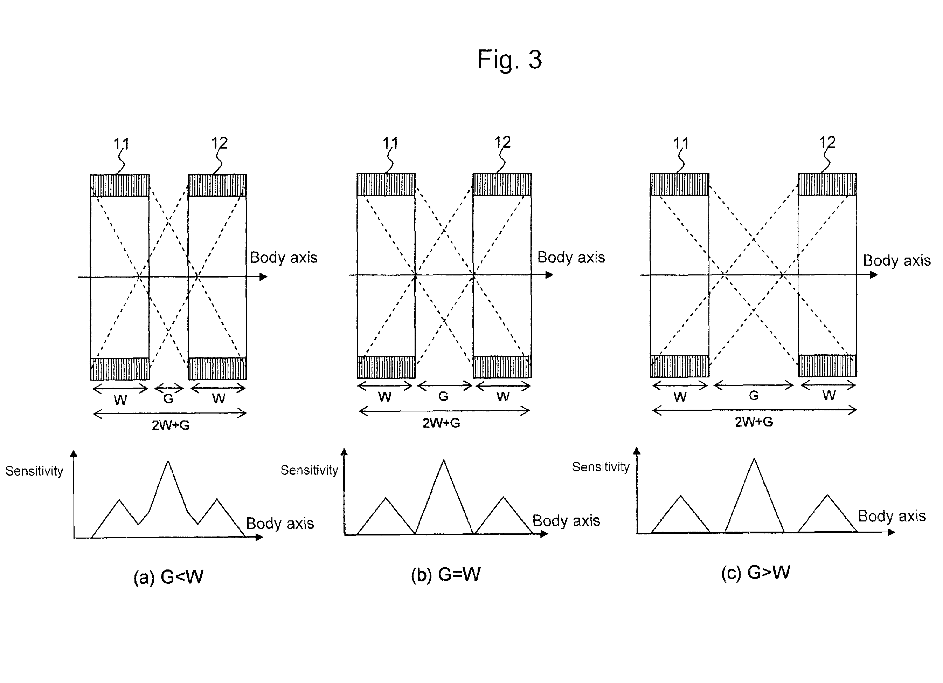

Here, as shown in Fig . 3, whenadimensionofeachof the detectors 11, 12 in the body axis direction (also referred to as a width) is given as W and a dimension of the open region in the body axis direction (also referred to as a clearance) is given as G, a field-of-view in the body axis direction is given as 2W + G. As shown in Fig. 3(c), when an open-region clearance G is in excess of W, a region which can be imaged is discontinued in the body axis direction. Therefore, as shown in Fig. 3(b), an upper limit of the open region clearance G for obtaining a field-of-view continuing in the body axis direction is given as W. However, the sensitivity is concentrated at the center of the open region to result in a drastic reduction in sensitivity in the periphery of the open region. In order to suppress a drastic reduction in sensitivity at both ends of the open region, as shown in Fig. 3(a), it is necessary to set G to be smaller than W, which, however, reduces an open-region clearance and a field-of-view in the body axis direction (refer to the above document).

As described so far, in the open-type PET scanner previously proposed by the applicant, there is a problem that the sensitivity is concentrated at the center of the open region to result in a drastic reduction in sensitivity in the periphery of the open region. Therefore, in order to suppress the local reduction in sensitivity, it is necessary to expand W relatively with respect to G. Further, amaximumvalue of an open-region clearance and that of a field-of-view in the body axis direction are limited respectively to W and 3W. Thus, in order to further expand the open-region clearance and the field-of-view in the body axis direction, it is necessary to expand W itself. However, in each case, a problem that an increase in the number of detectors constituting one multiple ring-type detector makes the scanner more expensive and more complicated has still been found.

In a conventional PET scanner which is not of an open-type, with an aim to measure a wider field-of-view by using detector rings with a limited field-of-view, a method in which radiation measurement is performed, with a bed or a PET scanner itself moved relatively has been adopted (refer to Japanese Published Unexamined Patent Application No. 2007-206090, Kitamura K., Takahashi S., Tanaka A., et al: 3D continuous emission and spiral transmission scanning for high-throughput whole-body PET. Conf. Rec. IEEE NSS & MIC. M3-2, 2004). This method did not solve the problems of an open-type PET scanner.

### Disclosure of the Invention

The present invention has been made for solving the above-described conventional problems, an object of which is to suppress a local reduction in sensitivity as well as expand an open-region clearance between detector rings and a field-of-view in the body axis direction without increasing the number of detectors.

The present invention is to provide an open-type PET scanner in which radiation measurement is performed while at least some of the detector rings are relatively moved with respect to a subject in the body axis direction, thereby dispersing coincidence lines in an open region and suppressing a local reduction in sensitivity.

Here, that an open-region clearance which undergoes moment to moment change with the lapse of time by relative movement of the detector rings may be always overlapped at least partially during the radiation measurement.

Further, at least one open-region clearance with an invariable width may be positionally fixed with respect to a subject during the radiation measurement.

Further, the detector rings may be optimized in constitution, moving direction and moving speed, thus making it possible to reduce the variation in distribution of sensitivity as well as expand an open-region clearance and a field-of-view in the body axis direction.

Further, a plurality of detector rings may be brought closer to each other and/or made apart from each other.

Further, a plurality of detector rings may be moved, with a certain distance kept in the body axis direction.

Still further, a plurality of detector rings may be brought closer to each other and/or made apart from each other, while they are allowed to move in the same direction in a one-way manner or in a reciprocating manner.

In addition, a moving detector ring may be housed inside a gantry and the gantry is fixed itself with respect to a subject during radiation measurement.

The present invention is an open-type PET scanner which includes a plurality of detector rings having multiple rings arrayed in the body axis direction, in which radiation measurement is performed while at least one detector ring is relatively moved with respect to a subject in the body axis direction, thereby dispersing coincidence lines which cut across an open region.

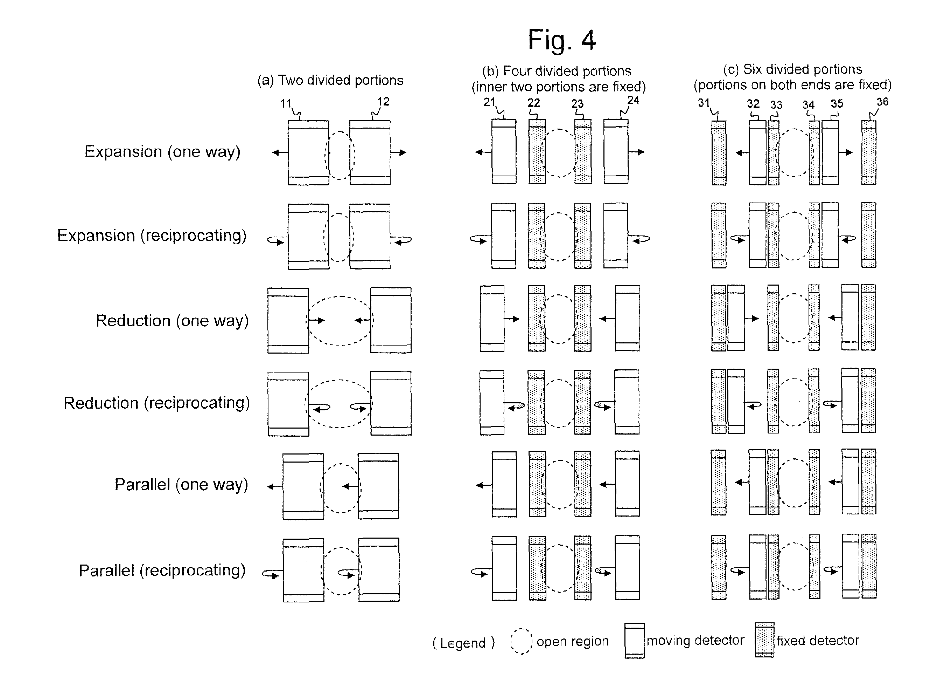

Fig. 4 illustrates representative patterns on a constitution and a moving direction of the detector ring. Fig. 4(a) shows a constitution in which both of the two divided detector rings 11, 12 are made movable to give an open region between them. Fig. 4 (b) shows a constitution in which the detector rings which have been respectively divided into two portions (21, 22), (23, 24) are arranged laterally, the two external detector rings 21, 24 are made movable, and a space held between the two central fixed detector rings 22, 23 is given as an open region. Fig. 4(c) shows a constitution in which the detector rings which have been respectively divided into three portions (31, 32, 33), (34, 35, 36) are arranged laterally, the two external detector rings 31, 36 and the two central detector rings 33, 34 are fixed, and the remaining two detector rings 32, 35 are made movable. In this instance, a space held between the central fixed detector rings 33, 34 is given as an open region.

In each case, as shown in Fig. 4, a moving direction of the movable detector rings can be set by a method of moving the detector rings so as to make them apart (expanding a clearance), a method of moving the detector rings so as to bring them closer (reducing a clearance), a method of shifting them, with a certain distance kept (parallel) and a method of moving them in the same direction to expand or reduce a clearance. They are allowed to move in a one-way manner or in a reciprocating manner.

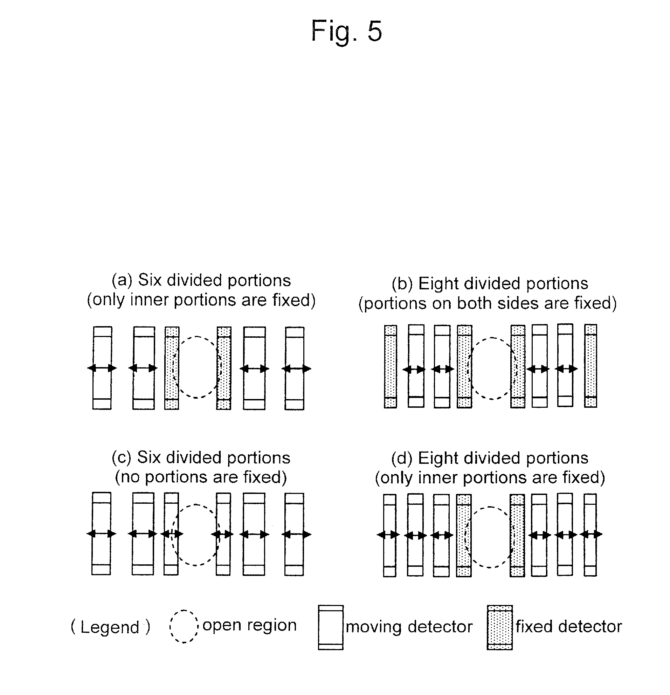

Fig. 5 shows constitution examples in which the movable detector rings are increased in number or divided into lager number of portions.

Fig. 4 and Fig. 5 show examples where a bed is fixed and only the detector rings are moved. On the other hand, the bed may be moved to have a relative movement with respect to the detector rings.

Measurement can be made while continuously moving, or repeating steps of stopping to make measurement and moving with small interval. Further, the movement speed and/or step interval may be changed.

An open-type PET scanner is able to reduce stress resulting from visual compression that a subject will experience when the head is subjected to a PET examination. Further, the scanner is expected to give PET diagnosis to a patient under treatment which would be otherwise impossible, for example, by realizing cancer treatment from an open space.

The present invention is able to suppress a local reduction in sensitivity in an open region, thus making it possible to enhance the image quality of an open space as a whole including the periphery of the open space, in addition to the center thereof.

The present invention is able to expand a field-of-view range without changing the total number of detectors, thereby providing a PET scanner capable of making a systemic diagnosis at once at a relatively lower price. A PET scanner capable of providing a systemic and simultaneous field-of-view has been considered indispensable in promoting a micro-dosing study which has gained attention as a method for effectively developing pharmaceuticals. The micro-dosing study is a method for selecting compounds of development candidates exhibiting optimal pharmacokinetics in humans by administering a compound in a trace amount at an earlier stage of the development in order to develop new pharmaceuticals effectively.

### Brief Description of the Drawings

Fig. 1(a) covers a perspective view and a cross sectional view showing a constitution of a conventional general PET scanner and Fig. 1(b) covers a perspective view and a cross sectional view showing a constitution of the open-type PET scanner previously proposed by the applicant.
Fig. 2 is a cross sectional view showing the principal of image reconstruction in an open-type PET scanner.
Fig. 3 covers cross sectional views and graphs showing relationships between an open region clearance and the sensitivity in an open-type PET scanner.
Fig. 4 covers drawings showing constitution examples of the present invention.
Fig. 5 also covers other constitution examples.
Fig. 6(a) is a drawing showing a condition of approaching shift, Fig. 6(b) is a drawing showing a condition of parallel shift and Fig. 6(c) is a drawing showing a condition of unilateral shift in constitution examples of the present invention.
Fig. 7(a) is a drawing showing a distribution of sensitivity and Fig. 7(b) is a drawing showing reduction/expansion patterns of an open-region clearance in the approaching shift.
Fig. 8(a) is a drawing showing a distribution of sensitivity and Fig. 8(b) is a drawing showing patterns in the parallel shift.
Fig. 9(a) is a drawing showing a distribution of sensitivity and Fig. 9(b) is a drawing showing position coordinates on the body axis at the centers of detectors in the unilateral shift.
Fig. 10(a) is also a drawing showing another example of a distribution of sensitivity and Fig. 10(b) is a drawing showing another example of position coordinates on the body axis at the centers of detectors in the unilateral shift.
Fig. 11 is a drawing showing a constitution of an embodiment of the present invention.
Fig. 12 covers drawings showing an example of parameters optimized in the embodiment and a distribution of sensitivity.
Fig. 13 covers drawings showing Embodiment 1 of the present invention.
Fig. 14 covers drawings showing Embodiment 2 of the present invention.

### Best Mode for Carrying Out the Invention

Hereinafter, an explanation will be made in detail for embodiments of the present invention by referring to the drawings.

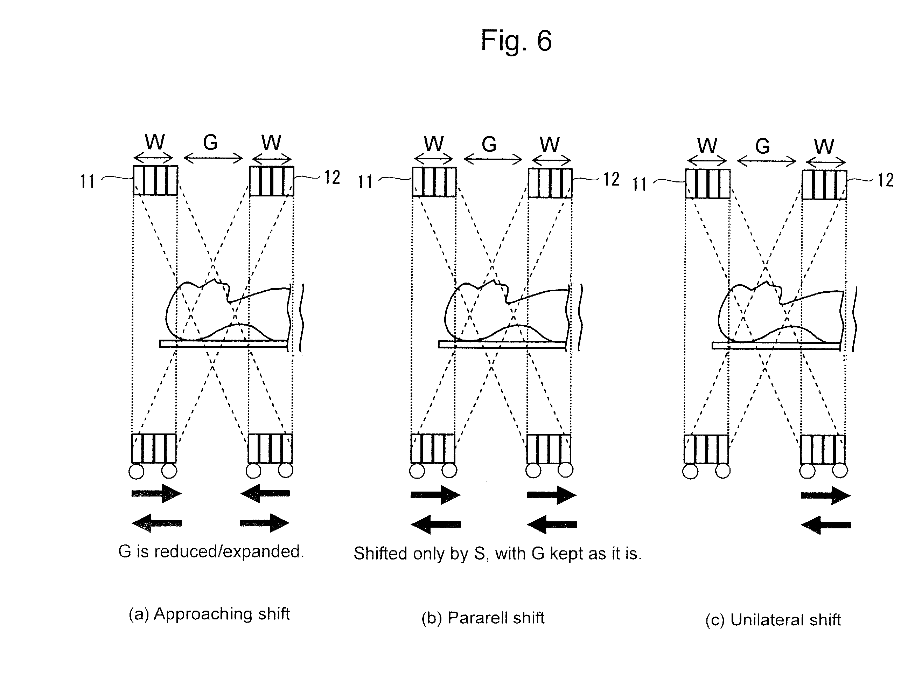

A simulation was conducted by using a computer in which on the basis of a commercially available PET scanner, as shown in Fig. 6, detector rings 11, 12 constituted with 32 detecting element rings (4.8 mm in width) having 576 detecting elements (scintillators) on a circumference the diameter of which is 827 mm were arranged apart laterally. A width W of the detector ring is 153.6 mm.

The moving direction was tested in a case where, as shown in Fig. 6(a), an open-region clearance G was reduced or expanded, in a case where, as shown in Fig. 6(b), detector rings on both sides were shifted by S, with the open-region clearance G kept constant, and in a case where, as shown in Fig. 6(c), the detector ring on one side only was shifted.

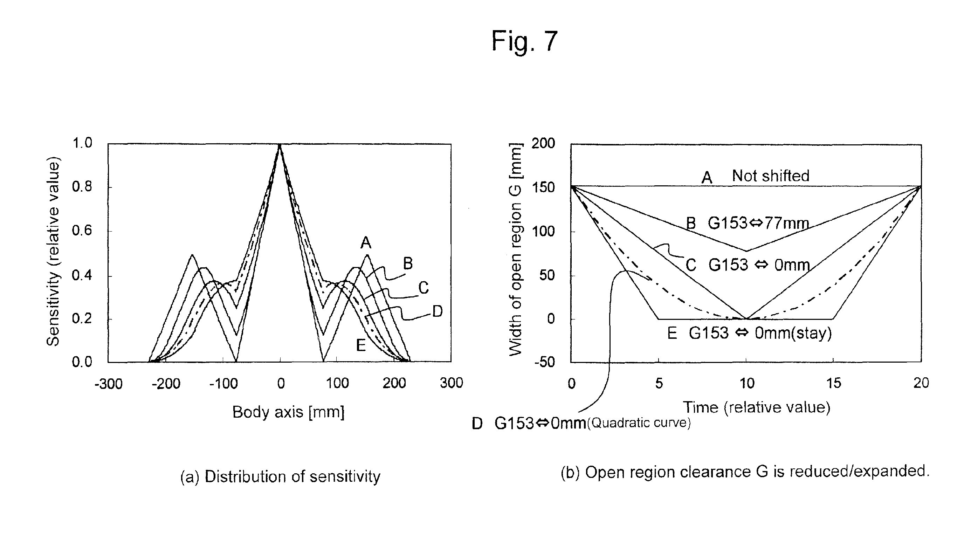

Fig. 7 shows the result obtained by trying the approaching shift. Fig. 7(a) shows the result of distribution of sensitivity obtained by five different moving methods from A to E. Fig. 7(b) illustrates temporal change in open-region clearance G. A is a conventional case where, with G = 153 mm kept as it is, no detector ring is moved, showing a drastic reduction in distribution of sensitivity. B and C are cases where after G is reduced at a constant speed from 153 mm to respectively 77 mm and 0 mm, G is again expanded up to 153 mm at a constant speed, showing that the reduction in sensitivity found in a conventional case is suppressed. E is a case where the moving speed is increased two times in the case of C and stopping movement is added under the condition of G = 0 mm, showing that the time of detector rings residing at the center of the scanner is increased to improve the characteristics of sensitivity. In addition, D is a case where the moving speed is increased or decreased in the case of C and expected to improve the characteristics of sensitivity as with the case of E and also expected to reduce mechanical burdens on folding back the detector rings. In each case, the approaching shift is characterized in that a peak value at the center of distribution of sensitivity is kept unchanged.

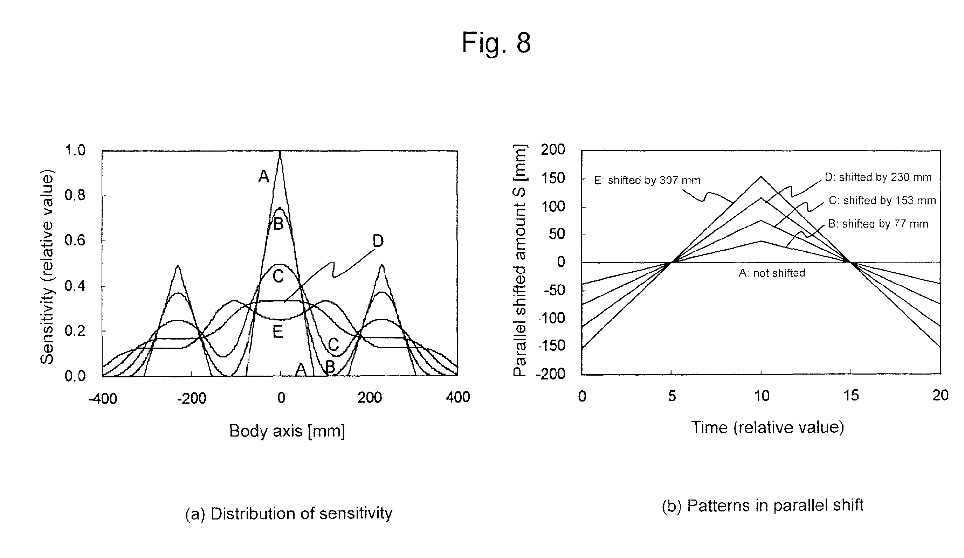

Fig. 8 shows the result obtained by trying the parallel shift, with G = 307 mm kept as it is. Fig. 8(a) shows the result of distribution of sensitivity obtained by five different moving methods from A to E. Fig. 8(b) illustrates patterns of the parallel shift. Detector rings are shifted at a constant moving speed in a reciprocating manner. A is a conventional case where, with G = 307 mm kept as it is, no detector ring is moved, showing a drastic reduction in distribution of sensitivity and the presence of a zero-sensitivity region. B through D are cases where the detector rings are shifted respectively only by 77 mm, 153 mm and 230 mm, showing that, with an increase in the shifted amount, a peak value of distribution of sensitivity is dispersed to increase the uniformity of distribution of sensitivity. E is a case where the detector rings are shifted up to 307 mm, showing that as compared with the case of D, a peak of sensitivity is newly invited.

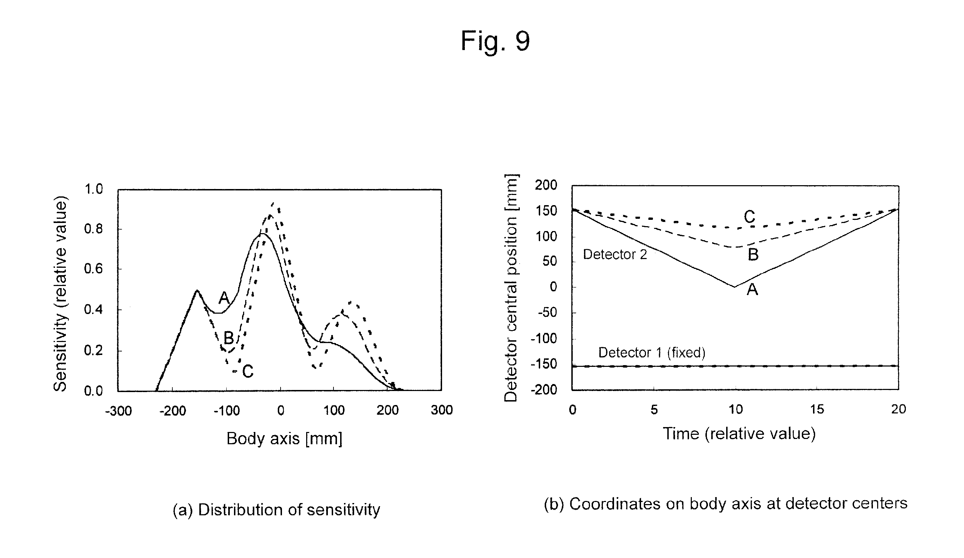

Fig. 9 shows the result obtained by trying the unilateral shift. Fig. 9(a) shows the result of distribution of sensitivity by three different moving methods from A to C. Fig. 9(b) illustrates position coordinates on the body axis at detector centers of detector rings (a detector 1 and a detector 2) on both sides. In each case, the detector 1 is fixed, only the detector 2 is made movable, and a clearance at the time of 0 is G = 153 mm. A through C are cases where the clearances are respectively reduced to 0 mm, 77 mm and 115 mm, and a peak of sensitivity of the detector 2 on the shifted side (on the right side in the drawing) is dispersed to suppress a local reduction in sensitivity in an open region.

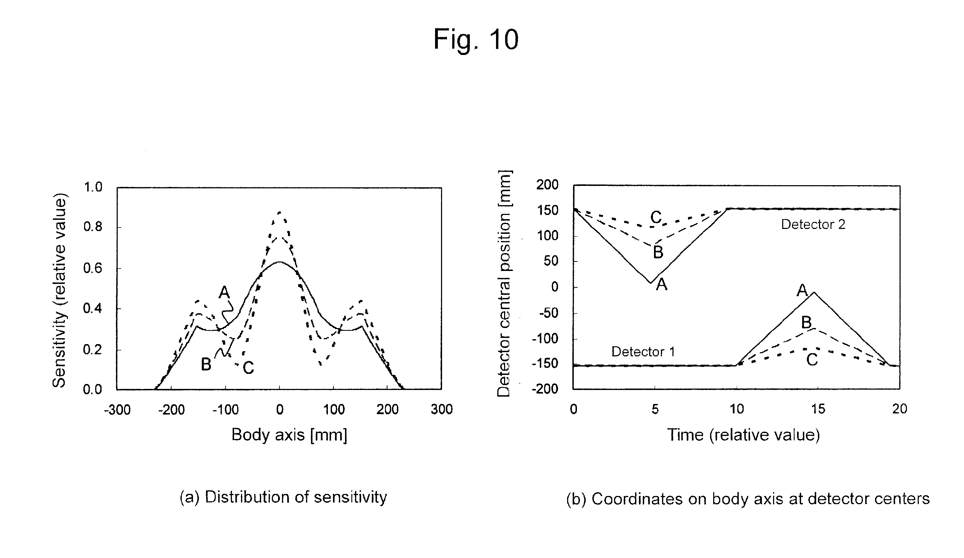

Fig. 10 shows the result obtained by exchanging a detector ring to be fixed in the unilateral shift. Fig. 10 (a) shows the result of distribution of sensitivity obtained by trying three different moving methods from A to C. Fig. 10(b) illustrates position coordinates on the body axis at the detector centers of the detector rings (a detector 1 and a detector 2) on both sides. In each case, the detector 1 was fixed and the detector 2 was made movable from the time 0 to 10, while the detector 2 was fixed and the detector 1 was made movable from the time 10 to 20. In the above case, a clearance at the time 0 was given as G = 153 mm, and the respective minimum clearances in A through C were given as 0 mm, 77 mm and 115 mm. It is found that apeak of sensitivity is dispersed to suppress a local reduction in sensitivity and the effect thereof is symmetrical and increased with an increase in the shifted amount.

In an open-type PET scanner having two-divided detector rings, the sensitivity is distributed so as to have, in addition to a central peak, peaks on both sides which are about half of the central peak. Since the approaching shift is able to offset a difference in sensitivity by shifting the peaks on both sides to the center, with the central peak kept as it is, this is a method for enhancing image quality by narrowing down to an open region. On the other hand, since the parallel shift is effective in offsetting a difference in sensitivity by cutting the respective peaks of sensitivity, this is a method for enhancing image quality not only at an open region but also in a field-of-view in the body axis direction as a whole. Thereby, it is right to say that the approaching shift is appropriate for fusion of diagnosis and medical treatment, while the parallel shift is appropriate for systemic and simultaneous field-of-view imaging.

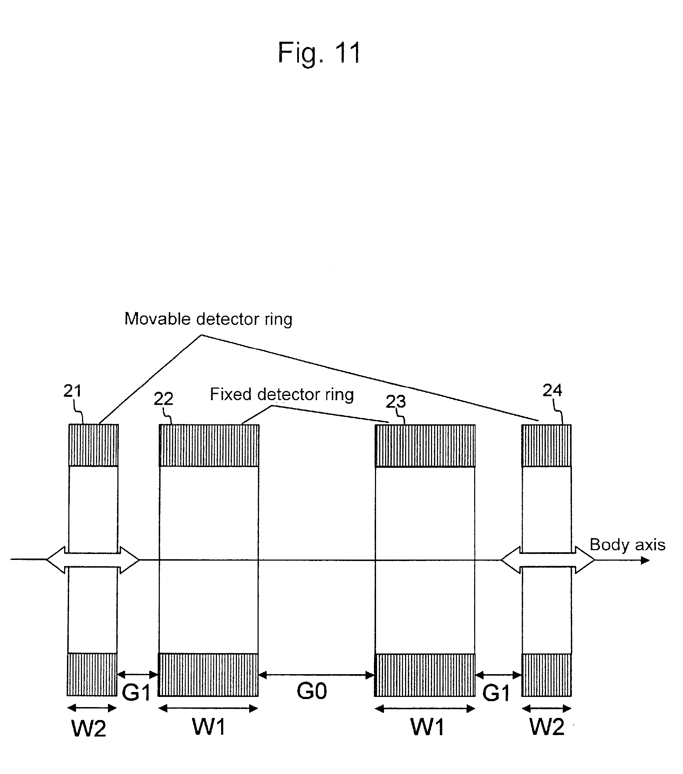

Next, there is shown an example where two-divided detector rings are arranged on both sides to give a total of four-divided detector rings, two inner detector rings are fixed while two external detector rings are made movable, thereby detectors are optimized in constitution, moving direction and moving amount. More specifically, as shown in Fig. 11, an open region clearance was given as G0, the inner fixed detector rings 22, 23 were given as W1 in width, the external movable detector rings 21, 24 arranged apart by clearance G1 from the fixed detector rings 22, 23 were given as W2 in width, and G1 was allowed to change from G1start to G1end. Then, under the conditions of G0 = 150 mm and W1 + W2 = 150 mm, obtained was a combination of W1, W2, G1start, and G1end to minimize a standard deviation (variation) of distribution of sensitivity within an open region.

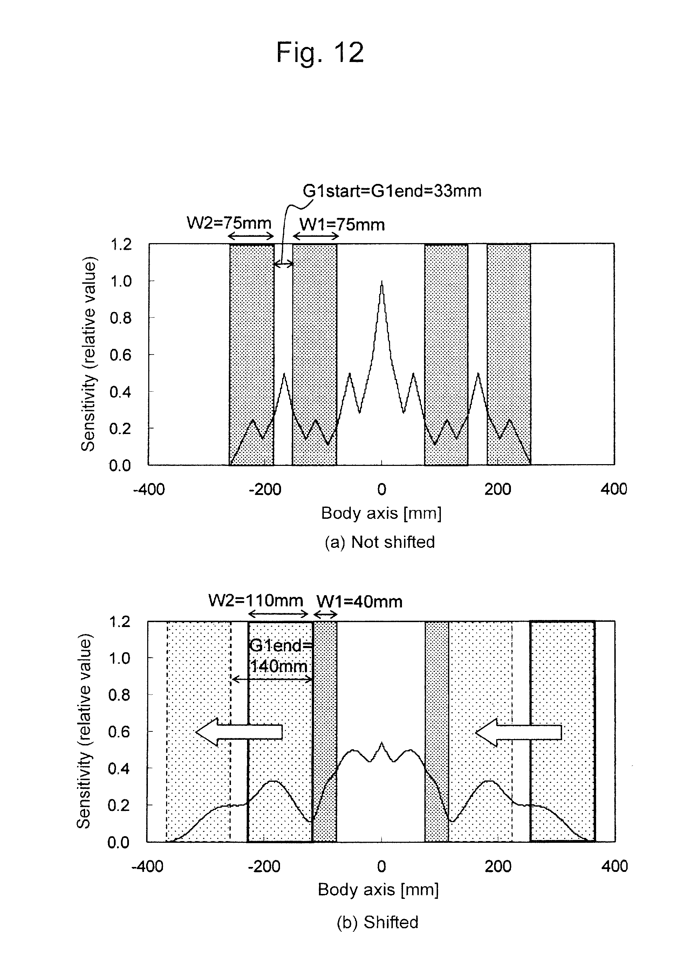

Fig. 12 shows optimized parameters and a distribution of sensitivity. Fig. 12(a) shows the result in which, as a reference, under the condition of not shifting a detector ring (G1start = Glend), W1, W2 and G1 are optimized. Although there is found no drastic reduction in sensitivity on both sides of the open region as shown in the distribution of sensitivity in Fig. 3(b), there are sharp peaks and valleys of sensitivity. On the other hand, Fig. 12(b) shows the result where a maximum value of G1end is set to be 140 mm, thereby optimizing parameters. It is found that a constitution in which the movable detector rings 21, 24 with the width of W2 = 110 mm are shifted parallel from G1start = 0 mm to G1end = 140 mm is able to minimize a variation of distribution of sensitivity in the open region. It is also found that the shift of detectors is effective not only in suppressing a local reduction in sensitivity but also in expanding a field-of-view in the body axis direction.

Next, an explanation will be made for an embodiment where detector shift according to the present invention is implemented. A gantry which houses internally the detector rings may be moved itself. However, since the gantry makes a relative shift with respect to a subject, it is difficult to secure sufficiently an open region immovable with respect to the subject. Further, there is needed attachment of a safety device for avoiding contact of the subject and/or operator with the gantry, which will make the constitution of the scanner more complicated.

Therefore, such a system is desirable that the gantry itself is kept fixed to the subj ect at least during measurement and a detector ring is moved inside the gantry.

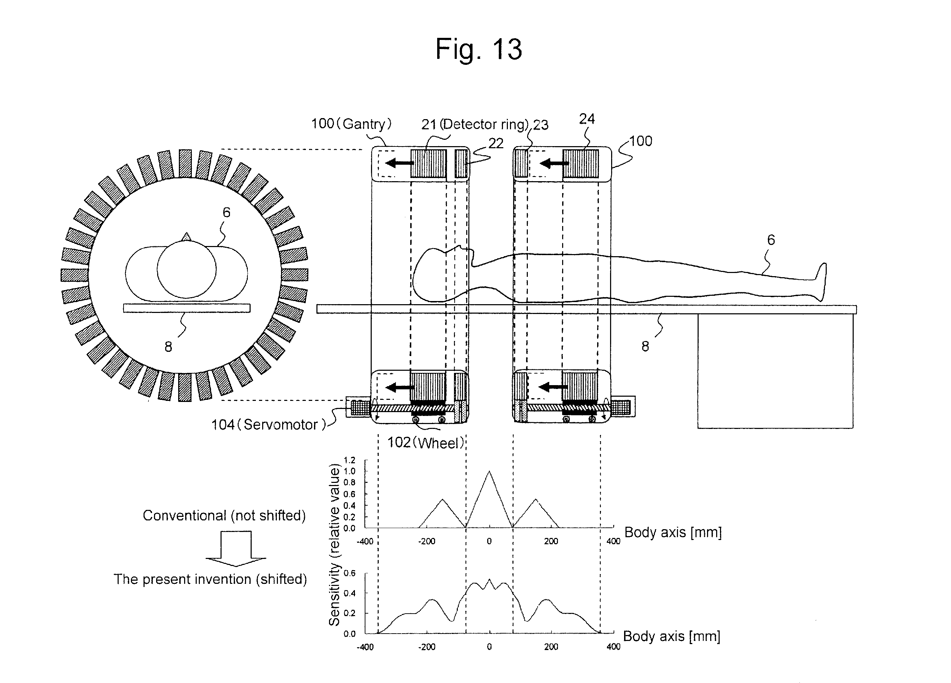

Fig. 13 shows Embodiment 1 where implemented is a system in which a detector ring is shifted inside a gantry 100 on the basis of the parameters optimized in Fig. 12(b). In this drawing, the reference numerals 102 and 104 depict respectively a wheel and a servomotor.

Not only is a certain open region secured, but also a safety device related to the movement of a detector ring can be simplified because no moving parts may be in contact with a subject.

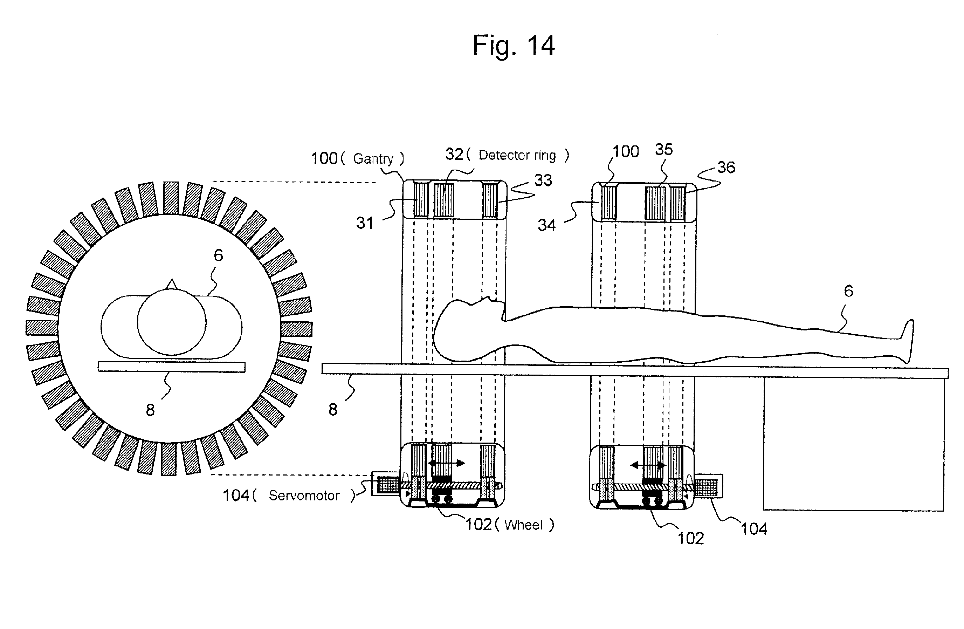

Fig. 14 shows Embodiment 2 in which implemented is a system where a detector ring is shifted inside the gantry 100 by referring, as an example, to a constitution where the detector ring is divided into six portions (three-divided detector rings are arranged on both sides), only detector rings 32 and 35 which are respectively a second and a fifth ring from the end (the left end in the drawing) are made movable.

In each of the above described embodiments, since the movable detector rings (21, 24) (32, 35) are housed inside the gantry 100 and the gantry is fixed, the safety is increased. In addition, the gantry can also be moved.

### Industrial Applicability

The present invention relates to an open-type PET scanner which includes a plurality of detector rings having multiple rings arranged in the body axis direction, and the scanner is able to suppress a local reduction in sensitivity as well as expand an open region clearance between detector rings and a field-of-view in the body axis direction without increasing the number of detectors.

## Claims

1. An open-type PET scanner which includes a plurality of detector rings having multiple rings arrayed in the body axis direction, wherein
radiation measurement is performed while at least one detector ring is relatively moved with respect to a subject in the body axis direction.

2. The open-type PET scanner according to claim 1, wherein
an open-region clearance which undergoes moment to moment change with the lapse of time by relative movement of the detector rings is always overlapped at least partially during radiation measurement.

3. The open-type PET scanner according to claim 1, wherein
at least one open-region clearance with an invariable width is positionally fixed with respect to a subject during radiation measurement.

4. The open-type PET scanner according to any one of claim 1 to claim 3, wherein the detector rings can be individually changed in moving direction and/or moving speed.

5. The open-type PET scanner according to claim 4, wherein
a plurality of detector rings are brought closer to each other and/or make apart from each other.

6. The open-type PET scanner according to claim 4, wherein
a plurality of detector rings are made to move, with a certain distance kept in the body axis direction.

7. The open-type PET scanner according to claim 4, wherein a plurality of detector rings are brought closer to each other and/or make apart from each other, while they are made to move in the same direction in a one-way manner or in a reciprocating manner.

8. The open-type PET scanner according to any one of claim 1 to claim 7, wherein a moving detector ring is housed inside a gantry and the gantry is fixed itself with respect to a subject during radiation measurement.
